# EUROPÄISCHE PATENTSCHRIFT

(11) **EP 1 720 866 B1**
(45) Veröffentlichungstag und Bekanntmachung des Hinweises auf die Patenterteilung: **03.08.2011**
(21) Anmeldenummer: 04804455.6
(22) Anmeldetag: 31.12.2004
(51) Int. Cl.: C07D 409/14

(54) **HERSTELLVERFAHREN**
PRODUCTION METHOD
PROCEDE DE PREPARATION

(30) Priorität: 15.01.2004 DE 102004002044
(43) Veröffentlichungstag der Anmeldung: 15.11.2006
(73) Patentinhaber: Bayer Schering Pharma Aktiengesellschaft, 13353 Berlin (DE)
(72) Erfinder: BERWE, Mathias, 45549 Sprockhövel (DE); THOMAS, Christian, 42111 Wuppertal (DE); REHSE, Joachim, 42799 Leichlingen (DE); GROTJOHANN, Dirk, 51377 Leverkusen (DE)
(86) Internationale Anmeldenummer: PCT/EP2004/014870
(87) Internationale Veröffentlichungsnummer: WO 2005/068456

(56) Entgegenhaltungen:
- WO-A-01/47919
- WO-A-2004/060887

## Beschreibung

Die vorliegende Erfindung betrifft ein Verfahren zur Herstellung von 5-Chlor-*N*-({(5*S*)-2-oxo-3-[4-(3-oxo-4-morpholinyl)-phenyl]-1,3-oxazolidin-5-yl}-methyl)-2-thiophencarboxamid ausgehend von 2-[(2*S*)-2-Oxiranylmethyl]-1*H*-isoindol-1,3(2*H*)-dion, 4-(4-Aminophenyl)-3-morpholinon und 5-Chlorthiophen-2-carbonylchlorid.

Die Verbindung 5-Chlor-*N*-({(5*S*)-2-oxo-3-[4-(3-oxo-4-morpholinyl),phenyl],1,3,oxazolidin,5-yl)-methyl)-2-thiophencarboxamid ist aus WO-A 01/47919 bekannt und entspricht der Formel (I)

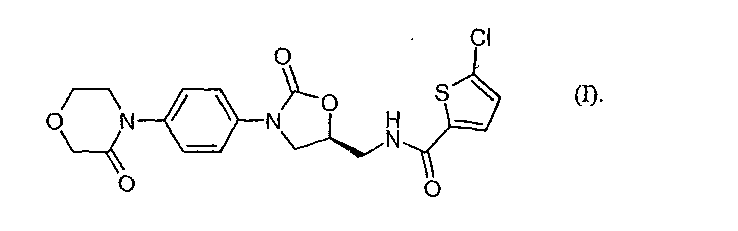

Die Verbindung der Formel (I) wirkt als Inhibitor des Blutgerinnungsfaktors Xa und kann als Mittel zur Prophylaxe und/oder Behandlung von thromboembolischen Erkrankungen insbesondere Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen eingesetzt werden.

In WO-A 01/47919 ist auch eine Methode zur Herstellung der Verbindung der Formel (I) im Gramm-Bereich, ausgehend von den gleichen Ausgangsverbindungen 2-[(2*S*)-2-Oxiranylmethyl]-1*H*-isoindol-1,3(2*H*)-dion (II), 4-(4-Aminophenyl)-3-morpholinon (III) und 5-Chlorthiophen-2-carbonylchlorid (IV), beschrieben:

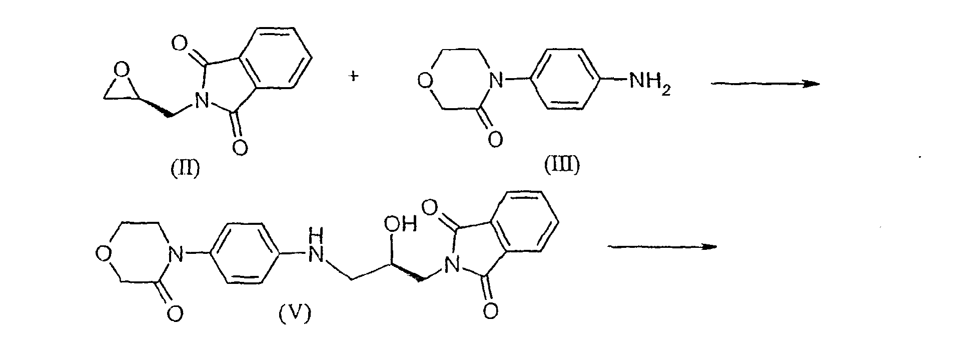

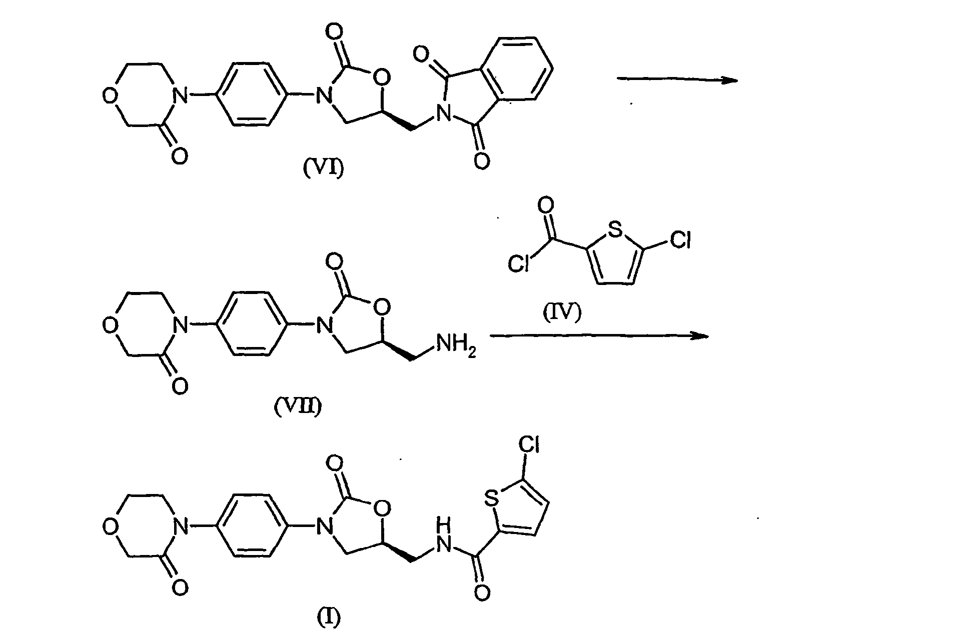

Hierbei wird 2-[(2*S*)-2,Oxiranylmethyl]-1*H*-isoindol-1,3(2*H*)-dion (II) mit 4-(4-Aminophenyl)-3-morpholinon (III) zu 2-((2*R*)-2-Hydroxy-3-{[4-(3-oxo-4-morpholinyl)phenyl]amino}propyl)-1*H-*isoindol-1,3(2*H*)-dion (V) umgesetzt. Anschließend wird (V) mit einem Phosgenäquivalent in 2-({(5*S*)-2-Oxo-3-[4-(3-oxo-4-morpholinyl)phenyl]-1,3-oxazolidin-5-yl}methyl)-1*H*-isoindol-1,3(2*H*)-dion (VI) überführt. Die Abspaltung der Phthalimidschutzgruppe liefert 4-{4-[(5*S*)-5-(Aminomethyl)-2-oxo-1,3-oxazolidin-3-yl]phenyl}morpholin-3-on (VII), das abschließend mit 5-Chlorthiophen-2-carbonylchlorid (IV) zu 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) umgesetzt wird.

Dieses aus WO-A 01/47919 bekannte Verfahren weist aber verschiedene Nachteile in der Reaktionsführung auf, die sich besonders ungünstig bei der Herstellung der Verbindung der Formel (I) in technischem Maßstab auswirken.

In DE 10300111.5 ist ein Alternativverfahren für die Synthese der Verbindung der Formel (I) ausgehend von 5-Chlorthiophen-2-carbonylchlorid (IV), (2*S*)-3-Amino-propan-1,2-diol Hydrochlorid (VIII) und 4-(4-Aminophenyl)-3-morpholinon (III) offenbart:

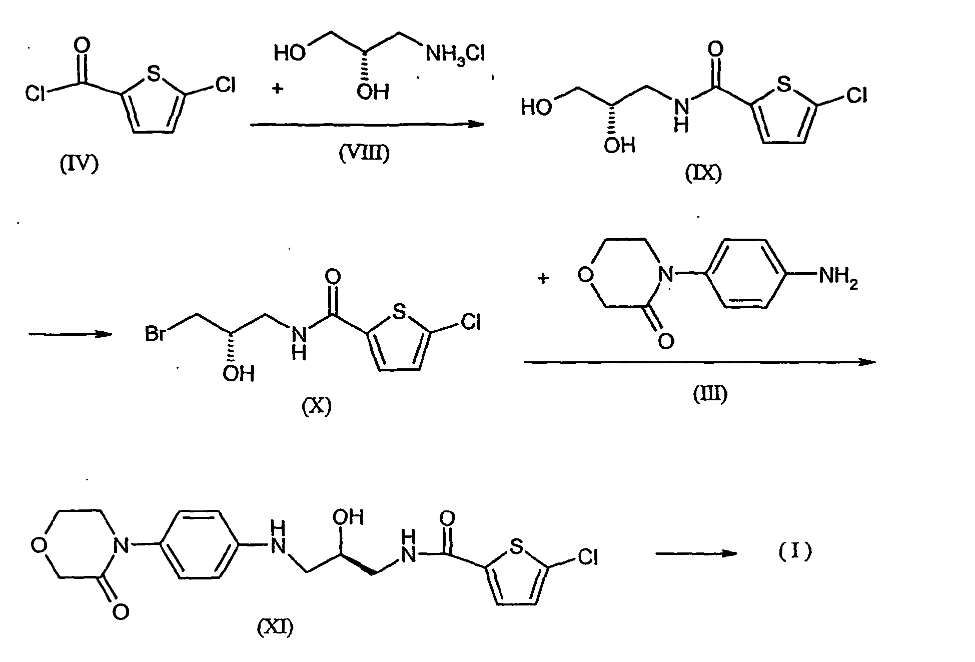

Hierbei wird 5-Chlorthiophen-2-carbonylchlorid (IV) mit (2*S*)-3-Amino-propan-1,2-diol Hydrochlorid (VIII) zu 5-Chlorthiophen-2-carbonsäure-((*S*)-2,3-dihydroxy-propyl)-amid (IX) umgesetzt. Anschließend wird (IX) in 5-Chlorthiophen-2-carbonsäure-((*S*)-3-brom-2-hydroxy-propyl)-amid (X) überführt, das dann mit 4-(4-Aminophenyl)-3-morpholinon (III) zu 5-Chlorthiophen-2-carbonsäure,{(*R*)-2-hydroxy-3-[4-(3-oxo-morpholin-4-yl)-phenylamino]-propyl}-amid (XI) umgesetzt wird. Abschließend wird (XI) mit Phosgen oder einem Phosgenäquivalent zu 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) umgesetzt.

Diese Altemativsynthese erlaubt eine Durchführung in technischem Maßstab, allerdings werden teilweise toxische Lösungsmittel oder Reagenzien verwendet. Dies ist *per se* von Nachteil, darüber hinaus müssen diese toxischen Substanzen aus dem Endprodukt (I) bis unterhalb die jeweils im Produkt aus regulatorischen Gründen zulässige Höchstgrenze entfernt werden, was einen zusätzlichen Aufwand bedeutet.

Daraus ergibt sich die Aufgabe der vorliegenden Erfindung, ein vereinfachtes Verfahren zur Herstellung der Verbindung (I) in technischem Maßstab unter Vermeidung toxischer Lösungsmittel oder Reagenzien insbesondere in den letzten Verfahrensschritten bereitzustellen.

Überraschenderweise wurde nun gefunden, dass sich durch Modifikation bestimmter Reaktionsparameter bei der aus WO-A 01/47919 bekannten Synthese die Verbindung der Formel (I) auch in größeren Mengen in guter Ausbeute und Reinheit herstellen lässt.

Gegenstand der vorliegenden Erfindung ist somit ein Verfahren zur Herstellung von 5-Chlor-*N-*({(5*S*)-2-oxo-3-[4-(3-oxo-4-morpholinyl)-phenyl]-1,3-oxazolidin-5-yl}-methyl)-2-thiophencarboxamid der Formel (I) durch Umsetzung von 4-{4-[(5*S*)-5-(Amiomethyl)-2-oxo-1,3-oxazolidin,3-yl]phenyl}morpholin-3-on (VII) Hydrochlorid mit 5-Chlorthiophen-2-carbonylchlorid (IV), dadurch gekennzeichnet, dass die Umsetzung in einem Lösungsmittel, ausgewählt aus der Gruppe von Ether, Alkohol, Keton und Wasser oder in einem Gemisch davon unter Verwendung einer anorganischen Base durchgeführt wird.

Als geeignete Lösungsmittel seien beispielhaft und vorzugsweise genannt: Ether wie Tetrahydrofuran, Dioxan, Diisopropylether oder Methyl-tert.-butylether; Alkohole wie Methanol, Ethanol, iso-Propanol, n-Propanol, n-Butanol, iso-Butanol, sec. Butanol oder tert.-Butanol; Ketone wie Methyl-ethylketon, Methyl-isobutylketon oder Aceton oder Wasser oder Gemische aus zwei oder mehr der aufgeführten Lösungsmittel.

Besonders bevorzugt als Lösungsmittel sind Ketone oder Gemische von Ketonen mit Wasser, insbesondere Aceton oder vorzugsweise Gemische von Aceton mit Wasser.

Als geeignete anorganische Basen seien beispielhaft und vorzugsweise genannt: Alkali- (z.B. Natrium- und Kalium-) und Erdalkali- (z.B. Calcium- und Magnesium-) hydroxide, Alkali- und Erdalkalicarbonate oder Alkali- und Erdalkalihydrogencarbonate.

Besonders bevorzugt als anorganische Base sind Natriumhydroxid, Natriumcarbonat oder Natriumhydrogencarbonat, insbesondere Natriumcarbonat.

Die Umsetzung von Aminomethyloxazolidinon (VII) Hydrochlorid mit Chlorthiophencarbonsäurechlorid (IV) wird bevorzugt in einem Aceton/Wasser-Gemisch als Lösungsmittel unter Verwendung von Natriumcarbonat als Base durchgeführt.

Hierbei ist das Aceton/Wasser-Verhältnis über einen großen Bereich variierbar, vorzugsweise beträgt es 0,5 bis 1,5 (v/v), insbesondere 0,9 bis 1,1 (v/v).

Auf diese Weise kann zum einen das cancerogene Pyridin, das in dem in WO-A 01/47919 beschrieben Verfahren als Lösungsmittel und Base verwendet wird, vermieden werden. Außerdem kann erfindungsgemäß die technisch aufwendige chromatographische Reinigung des Produktes (I) umgangen werden.

Bei der Durchführung des erfindungsgemäßen Verfahrens wird vorzugsweise eine wässrige Natriumcarbonatlösung vorgelegt, in die zunächst Aceton und dann Aminomethyloxazolidinon (VII) Hydrochlorid und anschließend Chlorthiophencarbonsäurechlorid (IV) eingetragen werden. Die Zugabe der Reaktanden erfolgt vorzugsweise bei einer Temperatur zwischen 0 und 20°C, insbesondere zwischen 10 und 15°C. Nach erfolgter Zugabe wird der Reaktionsansatz dann bei einer Temperatur zwischen 40 und 55°C, vorzugsweise bei ca. 50°C nachgerührt. Nach Abkühlung auf Raumtemperatur kann das Produkt dann in einfacher Weise durch Filtration isoliert werden.

In einer bevorzugten Ausführungsform der vorliegenden Erfindung wird das durch die oben beschriebene Filtration erhaltene Rohprodukt der Verbindung der Formel (I) in einem anschließenden Schritt zur weiteren Reinigung aus Essigsäure umkristallisiert.

Die Herstellung von Aminomethyloxazolidinon (VII) erfolgt, wie bereits auch in WO-A 01/47919 offenbart, durch Abspaltung der Phthalimidschutzgruppe von Oxazolidinonmethylphthalimid (VI) mit Methylamin in Ethanol als Lösungsmittel. Nach erfolgter Umsetzung wird aber noch, anders als in WO-A 01/479I9 beschrieben, wässrige Salzsäure bei erhöhter Temperatur zum Reaktionsgemisch bis zu einem pH-Wert zwischen 1 und 4, bevorzugt zwischen 2 und 3 gegeben. Die Zugabe erfolgt bei erhöhter Temperatur, vorzugsweise bei einer Temperatur zwischen 50 und 60°C. Auf diese Weise wird Aminomethyloxazolidinon (VII) in einfacher Weise rein in Form seines Hydrochlorides isoliert, das hierbei kristallin und gut filtrierbar anfällt.

Das Verfahren gemäß WO-A 01/47919, bei dem das nach Einengen des Reaktionsgemisches erhaltene Rohprodukt Aminomethyloxazolidinon (VII) direkt in der weiteren Umsetzung mit Chlorthiophencarbonsäurechlorid (IV) eingesetzt wird, hat dagegen den Nachteil, dass die Nebenkomponenten dieser Reaktion, die im Rohprodukt von Aminomethyloxazolidinon (VII) enthalten sind, die anschließende Herstellung des Endproduktes (I) behindern und das Produkt (I) zusätzlich verunreinigen. Im Gegensatz dazu ermöglicht die Verwendung von erfindungsgemäß als Hydrochlorid in reiner Form in Substanz isoliertem Aminomethyloxazolidinon (VII) in der folgenden Umsetzung mit Chlorthiophencarbonsäurechlorid (IV) eine verbesserte Reaktionsführung, wobei unerwünschte Nebenreaktionen vermieden werden und ein reineres Produkt erhalten wird, so dass die aufwendige chromatographische Reinigung vermieden werden kann.

Die Herstellung von Oxazolidinonmethylphthalimid (VI) erfolgt, wie bereits auch in WO-A 01/47919 offenbart, durch Cyclisierung der Hydroxyaminoverbindung (V) mit einem Phosgenäquivalent, beispielhaft und vorzugsweise mit N,N-Carbonyldiimidazol. Im Unterschied zu den in WO-A 01/47919 offenbarten Reaktionsbedingungen wird die Umsetzung aber statt in Gegenwart von Dimethylaminopyridin als Katalysator und Tetrahydrofuran als Lösungsmittel erfindungsgemäß ohne Katalysator in N-Methylpyrrolidon oder Toluol, vorzugsweise in Toluol als Lösungsmittel durchgeführt. Dadurch ist es auch möglich, entstandenes Oxazolidinonmethylphthalimid (VI) statt durch aufwendige chromatographische Reinigung durch einfache Filtration zu isolieren.

Die Herstellung des Hydroxyamins (V) erfolgt, wie bereits auch in WO-A 01/47919 offenbart, durch Umsetzung von (S)-Epoxyphthalimid (II) mit Anilinomorpholinon (III) in wässrigem Ethanol als Lösungsmittel bei einer Reaktionstemperatur von 60°C. Anders als in WO-A 01/47919 offenbart, beträgt das Ethanol/Wasser Verhältnis (v/v) aber statt 9:1 erfindungsgemäß 1:1 bis 1:3, vorzugsweise 1:2 (v/v) und es ist nicht mehr erforderlich, das Edukt (II) nachzudosieren. Statt dessen wird der Reaktionsansatz zwischen 24 und 48 Stunden, vorzugsweise ca. 36 Stunden, bei einer Temperatur zwischen 55 und 65°C gerührt.

In einer bevorzugten Ausffihrungsform der vorliegenden Erfindung wird das Reaktionsgemisch nach ein bis zwei Stunden Reaktionsdauer mit Impfkristallen des Reaktionsprodukts (V) versetzt, so dass das Reaktionsprodukt auszukristallisieren beginnt.

In einer besonders bevorzugten Ausführungsform der vorliegenden Erfindung wird der Reaktionsansatz gegen Ende der Reaktionszeit unter Rückfluss erhitzt, wobei die Suspension erhalten bleibt, und anschließend wieder auf die Reaktionstemperatur zwischen 55 und 65°C abkühlt.

Dieses Aufheizen zum Rückfluss wird gegebenenfalls wiederholt, vorzugsweise wird insgesamt zweimal aufgeheizt.

Die Synthese der Ausgangsverbindung (S)-Epoxyphthalimid (II) ist beispielsweise in [A. Gutcait et al. Tetrahedron Asym. 1996, 7, 1641] beschrieben. Außerdem ist die Substanz kommerziell erhältlich, beispielsweise bei der Firma Daiso Ltd., Japan.

Die Synthese der Ausgangsverbindung Anilinomorpholinon (III) ist beispielsweise in WO-A 01/47919, Seite 55 bis 57 oder in DE 10342570.5 ausführlich beschrieben.

Die einzelnen Stufen des erfindungsgemäßen Verfahrens können bei normalem, erhöhtem oder bei erniedrigtem Druck durchgeführt werden (z.B. von 0,5 bis 5 bar). Sofern nicht anders angegeben arbeitet man im allgemeinen bei Normaldruck.

Die Erfindung wird nachstehend durch ein bevorzugtes Ausführungsbeispiel näher erläutert, auf welches sie jedoch nicht eingeschränkt ist. Soweit nicht anders angegeben, beziehen sich alle Mengenangaben auf Gewichtsprozente.

### Synthese von 5-Chlor-N-({(5S)-2-oxo-3-[4-(3-oxo-4-morpholinyl)-phenyl]-1,3-oxazolidin-5-yl}-methyl)-2-thiophencarboxamid (I)

### a) 2-((2R)-2-Hydroxy-3-{[4-(3-oxo-4-morpholinyl)phenyl]amino}propyl)-1H-isoindol-1,3(2H)-dion (V)

1173 g 2-[(2*S*)-2-Oxiranylmethyl]-1*H*-isoindol-1,3(2*H*)-dion (II) und 4-(4-Aminophenyl)-3-morpholinon (III) werden bei 20°C mit 6,7l Wasser und 14,4l Ethanol versetzt. Die Suspension wird auf 58 bis 60°C erwärmt und die entstandene Lösung 36 Stunden nachgerührt. Nach 2 Stunden wird der Reaktionsansatz mit 5 g kristallinem 2-((2*R*)-2-Hydroxy-3-{[4-(3-oxo-4-morpholinyl)phenyl]amino}propyl)-1*H*-isoindol-1,3(2*H*)-dion (V) versetzt, woraufhin die Kristallisation des Produktes beginnt. Nach Abkühlen auf 26°C wird das ausgefallene Reaktionsprodukt abgesaugt, mit Ethanol gewaschen und dann getrocknet.

Ausbeute: 1522 g; entspricht 81,4 % der Theorie.

Schmelzpunkt: 215°C

### b) 2-({(5S)-2-Oxo-3-[4-(3-oxo-4-morpholinyl)phenyl]-1,3-oxazolidin-5-yl}methyl)-1H-isoindol-1,3(2H)-dion (VI)

2641 g 2-((2*R*)-2-Hydroxy-3-{[4-(3-oxo-4-morpholinyl)phenyl]amino}propyl)-1*H*-isoindol-1,3(2*H*)-dion (V) werden in 22 1 Toluol suspendiert und bei 19°C mit 1300 g N,N-Carbonyldiimidazol versetzt. Der Reaktionsansatz wird anschließend eine Stunde unter Rückfluss erhitzt und dann bei 60°C mit 4,5 1 Ethanol versetzt. Nach Abkühlen auf 25 bis 30°C wird das ausgefallene Reaktionsprodukt abgesaugt, mit Ethanol gewaschen und dann getrocknet.

Ausbeute: 2756 g; entspricht 97,9 % der Theorie.

Schmelzpunkt: 220,5°C

### c) 4-{4-[(5S)-5-(Aminomethyl)-2-oxo-1,3-oxazolidin-3-yl]phenyl}morpholin-3-on (VII)

1360 g 2-({(5*S*)-2-Oxo-3-[4-(3-oxo-4-morpholinyl)phenyl]-1,3-oxazolidin,5-yl}methyl)-1*H*-isoindol-1,3(2*H*)-dion (VI) werden bei 22°C in 10,2 1 Ethanol suspendiert und mit 1103 g Methylaminlösung (40%ig in Wasser) versetzt. Der Reaktionsansatz wird anschließend auf 60 bis 63°C erwärmt und die entstandene Lösung 2 Stunden bei dieser Temperatur gerührt. Nach Abkühlen auf 55 bis 60°C wird mit insgesamt 2348 g Salzsäurelösung (20 %ig in Wasser) bis zu einem pH-Wert von 2,7 versetzt, woraufhin die Kristallisation des Produktes beginnt. Nach Abkühlen auf 20°C wird das ausgefallene Reaktionsprodukt abgesaugt, mit Methanol gewaschen und dann getrocknet.

Ausbeute: 875 g; entspricht 82,7 % der Theorie.

Schmelzpunkt: Zersetzung oberhalb 280°C

¹H NMR (300 MHz, d₆-DMSO): 3.25 (m, 2H), 3.72 (m, 2H), 3.98 (m, 3H), 4.42 (m, 3H), 4.97 (m, 1H), 7.42 (d, 2H, *J* = 9.0 Hz), 7.57 (d, 2H, *J* = 9.0 Hz), 8.44 (s (br.), 3H) ppm.

### d) 5-Chlor-N-({(5S)-2-oxo-3-[4-(3-oxo-4-morpholinyl)-phenyl]-1,3-oxazolidin-5-yl}-methyl)-2-thiophencarboxamid (I)

### 1. Schritt: 5-Chlorthiophen-2-carbonylchlorid (IV)

3,00 kg 5-Chlorthiophen-2-carbonsäure (kommerziell erhältlich) werden in 8,48 kg Toluol suspendiert und auf 75 bis 80°C erwärmt. Bei dieser Temperatur werden 2,63 kg Thionylchlorid über einen Zeitraum von 85 Minuten zugetropft, anschließend wird 30 Minuten bei 75 bis 80°C und dann bei Rückflusstemperatur bis zur Beendigung der Gasentwicklung nachgerührt. Nach dem Abkühlen wird das Reaktionsgemisch bei vermindertem Druck und sukzessiv zunehmender Innentemperatur (bis maximal 60°C) destillativ von überschüssigem Thionylchlorid und Toluol befreit, bis eine ca. 30 %ige Lösung des Säurechlorides in Toluol entstanden ist.

### 2. Schritt: 5-Chlor-N-({(5S)-2-oxo-3-[4-(3-oxo-4-morpholinyl)-phenyl]-1,3-oxazolidin-5-yl}-methyl)-2-thiophencarboxamid (I) - Rohprodukt

Zu einer Lösung von 464 g Natriumcarbonat in 5,95 1 Wasser werden bei 10°C sukzessive 1160 g 4-{4-[(5*S*)-5-(Aminomethyl)-2-oxo-1,3-oxazolidin-3-yl]phenyl}-morpholin-3-on (VII) Hydrochlorid, 350 ml Wasser und 2,7l Aceton gegeben. Bei 8 bis 12°C werden 2535 g 5-Chlorthiophen-2-carbonylchlorid (IV) (30 %ige Lösung in Toluol) und weitere 517 ml Toluol zugegeben. Der Reaktionsansatz wird dann auf 50°C erwärmt, mit 2700 ml Aceton versetzt und weitere 30 Minuten bei 50 bis 53°C nachgerührt. Nach Abkühlen auf 26°C wird das ausgefallene Reaktionsprodukt abgesaugt und mit Wasser und Aceton gewaschen.

Ausbeute: 1998 g lösungsmittelhaltiges Rohprodukt.

Die ermittelte Restfeuchte beträgt 24,3 %, was einen errechneten Trockengewicht von 1505 g oder 98,7 % d. Th. entspricht.

### 3. Schritt: 5-Chlor-N-({(5S)-2-oxo-3-[4-(3-oxo-4-morpholinyl)-phenyl]-1,3-oxazolidin-5-yl}-methyl)-2-thiophencarboxamid (I) - Umkristallisation

2120 g lösungsmittelhaltiges Rohprodukt (Restfeuchte 9,4 %) wird in 12 kg Essigsäure suspendiert und auf 110 bis 115°C erhitzt. Die entstandene Lösung wird 10 Minuten bei dieser Temperatur nachgerührt und dann nach Klärfiltration auf 20°C abgekühlt. Das ausgefallene Produkt wird abgesaugt, mit Essigsäure und Wasser gewaschen und dann getrocknet.

Ausbeute: 1818 g; entspricht 94,7 % der Theorie (bezogen auf das Trockengewicht vom Rohprodukt).

Schmelzpunkt: 230°C

## Patentansprüche

1. Verfahren zur Herstellung von 5-Chlor-*N*-({(5*S*)-2-oxo-3-[4-(3-oxo-4-morpholinyl)-phenyl]-1,3-oxazolidin-5-yl}-methyl)-2-thiophencarboxamid der Formel (I) durch Umsetzung von 4-{4-[(5*S*)-5-(Aminomethyl)-2-oxo-1,3-oxazolidin-3-yl]phenyl}morpholin-3-on (VII) Hydrochlorid mit 5-Chlorthiophen-2-carbonylchlorid (IV), **dadurch gekennzeichnet, dass** die Umsetzung in einem Lösungsmittel, ausgewählt aus der Gruppe von Ether, Alkohol, Keton und Wasser oder in einem Gemisch davon unter Verwendung einer anorganischen Base durchgeführt wird.

2. Verfahren nach Anspruch 1, **dadurch gekennzeichnet, dass** die Umsetzung in einem Keton oder einem Gemisch von Keton und Wasser als Lösungsmittel durchgeführt wird.

3. Verfahren nach Anspruch 1 oder 2, **dadurch gekennzeichnet, dass** die Umsetzung mit Natriumhydroxid, Natriumcarbonat oder Natriumhydrogencarbonat als anorganischer Base durchgeführt wird.

4. Verfahren nach einem der Ansprüche 1 bis 3, **dadurch gekennzeichnet, dass** die Umsetzung in einem Aceton/Wasser-Gemisch als Lösungsmittel unter Verwendung von Natriumcarbonat als Base durchgeführt wird.

5. Verfahren nach einem der Ansprüche 1 bis 4, **dadurch gekennzeichnet, dass** eine wässrige Natriumcarbonatlösung vorgelegt wird und die Zugabe der Reaktanden bei einer Temperatur zwischen 10 und 15°C erfolgt und der Reaktionsansatz dann bei 50°C nachgerührt wird.

6. Verfahren nach einem der Ansprüche 1 bis 5, **dadurch gekennzeichnet, dass** das so erhaltene Rohprodukt der Verbindung der Formel (I) in einem anschließenden Schritt aus Essigsäure umkristallisiert wird.

7. Verfahren nach einem der Ansprüche 1 bis 6, wobei Aminomethyloxazolidinon (VII) Hydrochlorid durch Abspaltung der Phthalimidschutzgruppe von Oxazolidinonmethylphthalimid (VI) mit Methylamin in Ethanol als Lösungsmittel hergestellt wird, **dadurch gekennzeichnet, dass** Aminomethyloxazolidinon (VII) als Hydrochlorid in Substanz isoliert wird.

8. Verfahren nach Anspruch 7, **dadurch gekennzeichnet, dass** nach erfolgter Umsetzung von Oxazolidinonmethylphthalimid (VI) mit Methylamin wässrige Salzsäure bei einer Temperatur zwischen 50 und 60°C zum Reaktionsgemisch bis zu einem pH-Wert zwischen 2 und 3 gegeben wird.

9. Verfahren nach einem der Ansprüche 7 oder 8, wobei Oxazolidinonmethylphthalimid (VI) durch Cyclisierung der Hydroxyaminoverbindung (V) mit einem Phosgenäquivalent hergestellt wird, **dadurch gekennzeichnet, dass** die Umsetzung in Toluol als Lösungsmittel durchgeführt wird.

10. Verfahren nach Anspruch 9, **dadurch gekennzeichnet, dass** Oxazolidinonmethylphthalimid (VI) durch Filtration isoliert wird.

11. Verfahren nach einem der Ansprüche 9 oder 10, wobei das Hydroxyamin (V) durch Umsetzung von (S)-Epoxyphthalimid (II) mit Anilinomorpholinon (III) in wässrigem Ethanol als Lösungsmittel hergestellt wird, **dadurch gekennzeichnet, dass** das Ethanol/Wasser Verhältnis 1:2 beträgt.

12. Verfahren nach Anspruch 11, **dadurch gekennzeichnet, dass** das Reaktionsgemisch nach ein bis zwei Stunden Reaktionsdauer mit Impfkristallen des Reaktionsprodukts (V) versetzt wird.

13. Verfahren nach Anspruch 12, **dadurch gekennzeichnet, dass** das Reaktionsgemisch gegen Ende der Reaktionszeit zweimal unter Rückfluss erhitzt und anschließend jeweils wieder auf die Reaktionstemperatur zwischen 55 und 65°C abgekühlt wird.

## Claims

1. Process for preparing 5-chloro-*N*-({(5*S*)-2-oxo-3-[4-(3-oxo-4-morpholinyl)phenyl]-1,3-oxazolidin-5-yl}methyl)-2-thiophenecarboxamide of the formula (I) by reacting 4-{4-[(5*S*)-5-(aminomethyl)-2-oxo-1,3-oxazolidin-3-yl]phenyl}morpholin-3-one (VII) hydrochloride with 5-chlorothiophene-2-carbonyl chloride (IV), **characterized in that** the reaction is carried out in a solvent selected from the group of ether, alcohol, ketone and water or in a mixture thereof with use of an inorganic base.

2. Process according to Claim 1, **characterized in that** the reaction is carried out in a ketone or a mixture of ketone and water as solvent.

3. Process according to Claim 1 or 2, **characterized in that** the reaction is carried out with sodium hydroxide, sodium carbonate or sodium bicarbonate as inorganic base.

4. Process according to any of Claims 1 to 3, **characterized in that** the reaction is carried out in an acetone/water mixture as solvent with use of sodium carbonate as base.

5. Process according to any of Claims 1 to 4, **characterized in that** an aqueous sodium carbonate solution is initially charged, and the reactants are added at a temperature between 10 and 15°C, and the reaction mixture is then stirred at 50°C.

6. Process according to any of Claims 1 to 5, **characterized in that** the crude product of the compound of the formula (I) obtained in this way is recrystallized from acetic acid in a subsequent step.

7. Process according to any of Claims 1 to 6, where aminomethyloxazolidinone (VII) hydrochloride is prepared by eliminating the phthalimide protective group from oxazolidinonemethylphthalimide (VI) with methylamine in ethanol as solvent, **characterized in that** aminomethyloxazolidinone (VII) is isolated as solid hydrochloride.

8. Process according to Claim 7, **characterized in that** reaction of oxazolidinonemethylphthalimide (VI) with methylamine is followed by addition of aqueous hydrochloric acid to the reaction mixture at a temperature between 50 and 60°C until the pH is between 2 and 3.

9. Process according to either of Claims 7 or 8, where oxazolidinonemethylphthalimide (VI) is prepared by cyclization of the hydroxyamino compound (V) with a phosgene equivalent, **characterized in that** the reaction is carried out in toluene as solvent.

10. Process according to Claim 9, **characterized in that** oxazolidinonemethylphthalimide (VI) is isolated by filtration.

11. Process according to either of Claims 9 or 10, where the hydroxyamine (V) is prepared by reacting (S)-epoxyphthalimide (II) with anilinomorpholinone (III) in aqueous ethanol as solvent, **characterized in that** the ethanol/water ratio is 1:2.

12. Process according to Claim 11, **characterized in that** seed crystals of the reaction product (V) are added to the reaction mixture after the reaction has lasted from one to two hours.

13. Process according to Claim 12, **characterized in that** the reaction mixture is heated under reflux twice towards the end of the reaction time and on each occasion is subsequently cooled again to the reaction temperature of between 55 and 65°C.

## Revendications

1. Procédé pour la préparation de 5-chloro-*N-*({(5*S*)-2-oxo-3-[4-(3-oxo-4-morpholinyl)-phényl]-1,3-oxazolidin-5-yl}-méthyl)-2-thiophènecarboxamide de formule (I) par mise en réaction de chlorhydrate de 4-{4-[(5*S*)-5-(aminométhyl)-2-oxo-1,3-oxazolidin-3-yl]-phényl}morpholin-3-one (VII) avec du chlorure de 5-chlorothiophène-2-carbonyle (IV), **caractérisé en ce qu'**on effectue la réaction dans un solvant, choisi dans le groupe constitué par un éther, un alcool, une cétone et l'eau ou dans un mélange de tels solvants, en utilisant une base inorganique.

2. Procédé selon la revendication 1, **caractérisé en ce qu'**on effectue la réaction dans une cétone ou un mélange de cétone et d'eau en tant que solvant.

3. Procédé selon la revendication 1 ou 2, **caractérisé en ce qu'**on effectue la réaction avec de l'hydroxyde de sodium, du carbonate de sodium ou de l'hydrogénocarbonate de sodium en tant que base inorganique.

4. Procédé selon l'une quelconque des revendications 1 à 3, **caractérisé en ce qu'**on effectue la réaction dans un mélange d'acétone-eau en tant que solvant et en utilisant comme base du carbonate de sodium.

5. Procédé selon l'une quelconque des revendications 1 à 4, **caractérisé en ce qu'**on dispose au préalable une solution aqueuse de carbonate de sodium et on effectue l'addition des partenaires réactionnels à une température comprise entre 10 et 15 °C et on poursuit ensuite l'agitation du mélange réactionnel à 50 °C.

6. Procédé selon l'une quelconque des revendications 1 à 5, **caractérisé en ce que** dans une étape subséquente on fait recristalliser dans de l'acide acétique le produit brut du composé de formule (I) ainsi obtenu.

7. Procédé selon l'une quelconque des revendications 1 à 6, dans lequel on prépare le chlorhydrate d'aminométhyloxazolidinone (VII) par élimination du groupe protecteur phtalimido d'oxazolidinone-méthylphtalimide (VI) avec de la méthylamine dans de l'éthanol en tant que solvant, **caractérisé en ce que** l'aminométhyloxazolidinone (VII) sous forme de chlorhydrate est isolée telle quelle.

8. Procédé selon la revendication 7, **caractérisé en ce qu'**une fois effectuée la réaction de l'oxazolidinone-méthylphtalimide (VI) avec la méthylamine on ajoute une solution aqueuse d'acide chlorhydrique à une température comprise entre 50 et 60 °C au mélange réactionnel jusqu'à un pH compris entre 2 et 3.

9. Procédé selon l'une quelconque des revendications 7 ou 8, dans lequel on prépare l'oxazolidinone-méthylphtalimide (VI) par cyclisation du composé hydroxyamino (V) avec un équivalent de phosgène, **caractérisé en ce que** la réaction est effectuée dans du toluène en tant que solvant.

10. Procédé selon la revendication 9, **caractérisé en ce que** l'oxazolidinone-méthylphtalimide (VI) est isolé par filtration.

11. Procédé selon l'une quelconque des revendications 9 ou 10, dans lequel on prépare l'hydroxylamine (V) par mise en réaction de (S)-époxyphtalimide (II) avec de l'anilinomorpholinone (III) dans de l'éthanol aqueux en tant que solvant, **caractérisé en ce que** le rapport éthanol/eau est égal à 1:2.

12. Procédé selon la revendication 11, **caractérisé en ce qu'**après une durée de réaction d'une à deux heures on ajoute au mélange réactionnel des cristaux germes du produit de réaction (V).

13. Procédé selon la revendication 12, **caractérisé en ce que** vers la fin du temps de réaction on chauffe deux fois au reflux le mélange réactionnel et ensuite chaque fois on le refroidit à nouveau jusqu'à la température de réaction comprise entre 55 et 65 °C.
